# EUROPEAN PATENT APPLICATION

(11) **EP 1 741 710 A1**
(43) Date of publication of application: **10.01.2007**
(21) Application number: 05734744.5
(22) Date of filing: 25.04.2005
(51) Int. Cl.: C07D 401/12, A61K 31/454, A61P 3/10, A61P 43/00, C07D 405/14, C07D 409/14, C07D 453/02

(54) **PYRROLIDINE DERIVATIVE**

(30) Priority: 27.04.2004 JP 2004130674
(71) Applicant: Astellas Pharma Inc., Tokyo 103-8411 (JP)
(72) Inventor: HAYAKAWA, Masahiko, c/o Astellas Pharma Inc., Tokyo 1038411 (JP); NAKANO, Ryosuke, c/o Astellas Pharma Inc., Tokyo 1038411 (JP); FURUTANI, Masako, c/o Astellas Pharma Inc., Tokyo 1038411 (JP)
(74) Representative: HOFFMANN EITLE
(86) International application number: PCT/JP2005/007770
(87) International publication number: WO 2005/116012

(57) **Abstract**

There is provided a novel pyrrolidine derivative represented by the formula (I) or a salt thereof having an excellent DPP-IV inhibitory activity and an excellent pharmacokinetic. On the basis of the action as such, the compound of the present invention is useful for treatment and/or prevention of insulin-dependent diabetes mellitus (type 1 diabetes mellitus) and particularly for non insulin-dependent diabetes mellitus (type 2 diabetes mellitus), insulin-resistant diseases, obesity, etc. 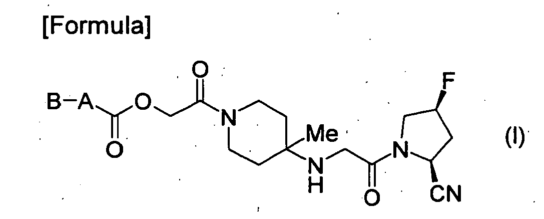 [In the formula, -A- is a single bond, -O-, -S-, -NH- or -N(lower alkyl)-; and -B is lower alkyl, lower alkenyl, lower alkynyl, cycloalkyl, aryl, non-aromatic hetero ring or aromatic hetero ring and each of those groups may be substituted.]

## Description

### [Technical Field]

The present invention relates to a novel pyrrolidine derivative or a salt thereof that is useful for a drug, especially as an inhibitor for dipeptidyl peptidase IV (hereinafter, referred to as "DPP-IV") and to a drug comprising the compound as an active ingredient.

### [Background Art]

Dipeptidyl peptidase-IV (DPP-IV) is a serine protease which recognizes and cleaves a sequence containing proline, hydroxyproline or alanine as the second member from N-terminal (H-Xaa-Pro, H-Xaa-Hyp or H-Xaa-Ala [Xaa is any amino acid]). Although its physiological role has not been completely clarified yet, it is likely to participate in regulation of biofunction by cleaving various physiologically active peptides (Non-Patent Document 1). Among the above, control of activity of hormone which is called incretin participating in suppression of an increase in postprandial blood glucose has been receiving public attention.
Incretin is a hormone which is secreted from intestinal tract after nutrient ingestion, acts on pancreatic β-cells and enhances insulin secretion by glucose stimulation whereby blood glucose is controlled. With regard to hormone showing an action of incretin *in vivo*, glucagon-like peptide and the like have been known and all of them have been known to be inactivated as a result of being cleaved by DPP-IV (Non-Patent Document 2).
From those facts, it is likely that a DPP-IV inhibitor enhances insulin secretion after meal due to prevention of inactivation of incretin and accordingly that hyperglycemia after meal noted in patients of diabetes mellitus is able to be corrected. In addition, incretin enhances insulin secretion which is dependent upon blood glucose concentration in humans and, therefore, it is expected to become a safe treating agent having no adverse action such as hypoglycemia which is noted in the conventional insulin secreting agents (Non-Patent Document 3).

Incidentally, it is common that a drug achieves its effect after administration when it comes into circulating blood flow and then reaches the action site. Therefore, for a purpose that a drug efficiently achieves its pharmaceutical effect, it is necessary that the administered drug reaches the circulating blood flow in a high rate. As to an index showing a transfer rate of the administered drug into circulating blood flow, bioavailability (BA) has been commonly used and, when the BA value is high, the administered drug is efficiently transferred into the circulating blood flow. In other words, it is expected that a drug having higher BA value is able to make the drug concentration at the action site adequate by less dose and shows higher pharmacological activity. Moreover, since unnecessarily high dose is not administered, it is expected that risk of adverse effect by an excessive administration of a drug becomes little and such a drug is believed to be highly safe and convenient.
Such safety and convenience of a compound having a high BA value are also the same as those in the case of a DPP-IV inhibitor and there has been a demand for creation of a DPP-IV inhibitor having a high BA value.

In the meanwhile, several pyrrolidine derivatives have been known as compounds having a DPP-IV inhibiting activity (Non-Patent Documents 1 to 8).
In WO 02/30890 (Patent Document 5), compounds represented by the formula (V) are disclosed and is mentioned that there is expected an effect in prevention and treatment of diabetes mellitus and in prevention and treatment of other diseases which are induced or exacerbated by the impaired glucose tolerance ability such as hyperinsulinemia and diabetic complication. 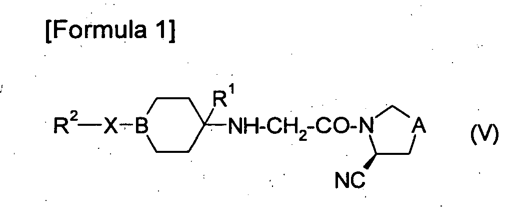 (With regard to the symbols in the formula, refer to the above WO gazette.)

In WO 2004/009544 (Patent Document 8), compounds represented by the formula (VIII) are disclosed and is mentioned to be effective in treatment and/or prevention for insulin-dependent diabetes mellitus (type 1 diabetes mellitus), non insulin-dependent diabetes mellitus (type 2 diabetes mellitus), insulin-resistant diseases, obesity, and the like. 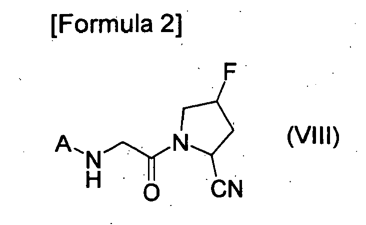 [Non-Patent Document 1] Mentlein, R., Regulatory Peptide, 1999, vol. 85, pages 9-24
[Non-Patent Document 2] Drucker, D. J., Diabetes, 1998, vol. 47, pages 159-69
[Non-Patent Document 3] Drucker, D. J., Diabetes Care, 2003, vol. 26, page 2929-40
[Patent Document 1] WO 98/19998
[Patent Document 2] WO 01/96295
[Patent Document 3] WO 00/34241
[Patent Document 4] WO 01/55105
[Patent Document 5] WO 02/30890
[Patent Document 6] WO 02/38541
[Patent Document 7] WO 03/002553
[Patent Document 8] WO 2004/009544

### [Disclosure of the Invention]

### [Problems that the Invention is to Solve]

Under such circumstances, there has been a brisk demand for a drug
having better DPP-IV inhibitory activity or, preferably, for a drug having higher BA value.

### [Means for Solving the Problem]

The present inventors have conducted intensive studies for compounds having a DPP-IV inhibitory activity which is expected to have the efficacy to insulin-dependent diabetes mellitus (type 1 diabetes mellitus), non insulin-dependent diabetes mellitus (type 2 diabetes mellitus), insulin-resistant diseases and obesity and found the novel pyrrolidine derivative or a salt of the present invention has an excellent inhibiting activity to DPP-IV and also shows an excellent pharmacokinetics whereupon the present invention has been achieved.
Thus, in accordance with the present invention, a pyrrolidine derivative represented by the following formula (I) or a pharmaceutically acceptable salt thereof useful as a DPP-IV inhibitor is provided. 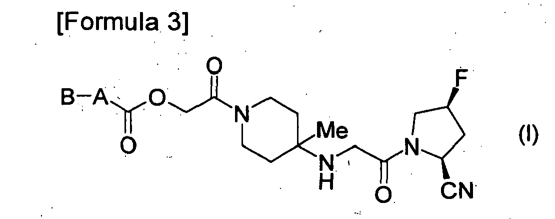 [In the formula, -A- is a single bond, -O-, -S-, -NH- or -N(lower alkyl)-; and -B is lower alkyl, lower alkenyl, lower alkynyl, cycloalkyl, aryl, non-aromatic hetero ring or aromatic hetero ring and each of those groups may be substituted.]
Among those compounds, a compound where -A- is a single bond is preferred; a compound where -A- is a single bond and -B is an optionally substituted lower alkyl or an optionally substituted aryl is more preferred; a compound where -A- is a single bond and -B is an optionally substituted lower alkyl is still more preferred; and a compound where -A- is a single bond and -B is methyl is particularly preferred.
As to other embodiments, a compound where -A- is -O- is preferred; a compound where -A- is -O- and -B is an optionally substituted lower alkyl or an optionally substituted aryl is more preferred; a compound where -A- is -O- and -B is an optionally substituted lower alkyl is still more preferred; and a compound where -A- is -O- and -B is methyl is particularly preferred.
In accordance with the present invention, a pharmaceutical composition comprising a compound represented by the above formula (I) as an active ingredient; a pharmaceutical composition among the above which is a treating agent for insulin-dependent diabetes mellitus (type 1 diabetes mellitus), non insulin-dependent diabetes mellitus (type 2 diabetes mellitus), insulin-resistant diseases and obesity; and/or a pharmaceutical composition which is a dipeptidyl peptidase IV inhibitor is/are provided.

### [Advantages of the Invention]

The compound of the present invention is a compound where, for the purpose of improvement of pharmacokinetics of the compound of Example 34 mentioned (hereinafter, referred to as "compound X") in the above WO 2004/009544 (Patent Document 8), hydroxyl group of the compound X is acylated, made into a carbonate, made into a thiocarbonate or made into a carbamate. In the above document, although there is a suggestion for a prodrug of the compound invented in the document, there is no specific disclosure for a compound in which hydroxyl group is acylated, made into a carbonate, made into a thiocarbonate or made into a carbamate.
Among the compounds for which pharmacological data in an evaluating test for duration of DPP-IV inhibitory activity to mice and rats are disclosed in the document, the compound X is a compound having the best duration of activity disclosed in the document. However, when a test was conducted according to the following test examples, the result was that BA of the compound X by oral administration is not so high and, therefore, there has been a demand for its improvement. Accordingly, an investigation was conducted for a compound where hydroxyl group of the compound X is acylated, made into a carbonate, made into a thiocarbonate or made into a carbamate concerning a DPP-IV inhibitory activity and BA. As a result, unlike common prodrugs, it was suggested not only that the compound of the present invention is converted to the compound X *in vivo* showing a DPP-IV inhibitory activity as the compounds X but also that the compound of the present invention itself shows an excellent DPP-IV inhibitory activity. With regard to BA, sum of blood levels of the compound of the present invention and the compound X which was produced by deacylation, decarbonation, dethiocarbonation or decarbamation after administration of the compound of the present invention significantly increased as compared with the case where the same dose of the compound X was administered whereby a significant improvement in BA was confirmed.

Pharmacological action of the compound of the present invention was confirmed by the following test examples.
(1) Test for determination of DPP-IV inhibitory activity
   Procedure of DPP-IV activity measurement is as follows. The reaction was performed in a 96-well plate.
   Reaction solutions (100 µl/well) obtained by adding a test substance at various concentrations to a solution containing 50 mM of Tris HCl, 150 mM of sodium chloride, a 0.1% triton X-1 00, 0.3 mU/ml of DPP-IV purified from pig kidney (Sigma), and 0.05 mM of Gly-Pro-AMC (Bachem) were incubated at a room temperature for 60 minutes, and then fluorescence intensities (Excitation: 355 nm, Emission: 460 nm) of the reaction solutions were measured (ARVO, Perkin Elmer). The data of 3 Wells under the same conditions were averaged.
   The inhibition in the test group, relative to the solvent-added group was calculated, and the IC₅₀ Value thereof was obtained through logistic analysis. The result is given in Table 1.

**[Table 1]**

| Tested Compound | IC₅₀ (nM) |
|---|---|
| Example 1 | 15.0 |
| Compound X | 15.0 |

As shown above, the compound of the present invention and the compound X showed a DPP-IV inhibitory activity of the same degree.

(2) Test for DPP-IV inhibiting activity duration in mice:
   Male ICR mice (Nippon SLC) were grouped into a test group and a control group of 5 subjects each: A test compound (10 mg/kg) was dissolved in purified water, and orally administered. Purified water alone was orally administered to the mice of the control group. One half and 12 hours after the administration, the blood was collected from each mouse through the orbital venous plexus thereof. The collected blood was immediately centrifuged to isolate the plasma, and the DPP-IV activity of the plasma was measured.
   The process of plasma DPP-IV activity determination was as follows: The reaction was performed in a 96-well plate. 5 µl of the collected plasma was added to an aqueous solution (95 µl/well) comprises 25 mM Tris-HCl, 140 mM sodium chloride, 10 mM potassium chloride, 1 % bovine serum albumin, and 0.01 mM Gly-Pro-AMC (Bachem), and incubated at room temperature for 20 minutes. The fluorescence intensity (excitation 355 nm/emission 460 nm) of each well was measured (ARVO, Perkin Elmer).
   The fluorescent intensity of the well, to which was added the plasma collected from the control group, was 100 %. Based on it, the DPP-IV activity of the plasma collected from the test compound-administered mice was calculated, and the activity difference between the control group and the test group was obtained. This indicated the inhibition in the test group. The result is given in Table 2.

**[Table 2]**

| Test Compound | Inhibitory Rate after 0.5 Hour from Administration (%) | Inhibitory Rate after 12 Hours from Administration (%) |
|---|---|---|
| Example 1 | 95 | 84 |
| Compound Y | 90 | 33 |

In the table, the compound Y is a compound of Example 4-17 mentioned in the above Patent Document 5 and has the following structure. 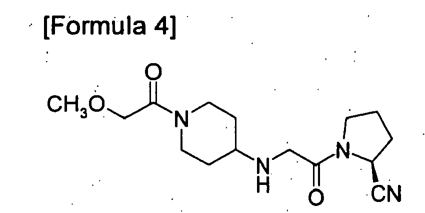 As shown above, the compound of the present invention had a good oral activity and its activity was found to have a sufficient duration of DPP IV inhibiting activity even after 12 hours from administration as compared with the compound Y which is a compound for comparison.

(3) PK test in rats
   Male SD rats of six weeks age (Nippon Clare) was fasted for one night, a test compound dissolved in distilled water was orally administered at the dose of 3 mg/kg and blood was collected from inferior vena cava under anesthetization after 0.25, 0.5, 1, 2, 4, 6, 10 and 12 hours from administration of the compound. In the meanwhile, a test compound dissolved in a physiological saline was administered from tail vein at the dose of 1 mg/kg and blood was similarly collected from inferior vena cava under anesthetization after 0.1, 0.25, 0.5, 1, 2, 4, 6 and 10 hours from administration of the compound. Each three animals were used for each point. Plasma was collected from the blood obtained by such a blood collection and concentration of the compound in plasma was measured using LC/MS/MS (2695, Waters and TSQ Quantum, ThermoFinnigan).
   On the basis of concentration of the compound in the plasma determined by the measurement, an area under curve (AUC) of the drug concentration in plasma vs. time was calculated and, from the ratio of AUC by oral administration to AUC by intravenous injection, bioavailability (BA) was calculated. With regard to the BA value by administration of the compound of the present invention, each of concentrations in plasma of the compound X and the compound of the present invention was calculated.

**[Table 3]**

| Compound Tested | Example 1 | | Compound X |
|---|---|---|---|
| Compound Measured | Example 1 | Compound X | Compound X |
| AOC_{0→∞} (ng·h/ml) | 43 | 522 | 138 |
| BA (%) | 7.7 | 59 | 16 |

As mentioned above, it was noted that the AUC calculated by the sum of the compound of the present invention and the compound X in plasma when the compound of Example 1 was orally administered increased to an extent of about fourfold as compared with the AUC value when the compound X was administered. Further, the BA value calculated by the sum concentrations of the compound of the present invention and of the compound X in plasma when the compound of Example 1 was orally administered calculated to be 68% whereby it was noted that it significantly increased as compared with 16% which was the BA value in case that the compound X was administered. Incidentally, as shown in the above (1), Example 1 and the compound X showed a DPP-IV inhibitory activity in the similar degree. From those results, it was confirmed that, as compared with the compound X, the compound of the present invention showed a high BA value or, in other words, it showed better pharmacokinetics.

(4) PK test in cynomolgus monkey
   Male cynomolgus monkeys of four year old (K. K. Cary; the country of origin: Vietnam) were fasted for 16 hours before administration (feed was given after 4 hours from administration), a test compound dissolved in distilled water was orally administered at a dose of 3 mg/kg and blood was collected from forearm cephalic vein after 0.25, 0.5, 1, 2, 4, 8 and 24 hours from administration of the compound. In addition, cynomolgus monkeys to which no drug was administered for 7 days after the oral administration were fasted during 16 hours before administration (feed was given after 4 hours from administration), a test compound dissolved in distilled water was administered at the dose of 1 mg/kg from forearm cephalic vein and blood was collected from the forearm cephalic vein after 0.1, 0.25, 0.5, 1, 2, 4, 8 and 24 hours from administration of the compound. For each of the administrations, three animals were used. Plasma was collected by centrifugal separation of the blood obtained by such a blood collection and concentration of the compound in the plasma was measured by LC/MS/MS (2695, Waters and TSQ Quantum, ThermoFinnigan).

**[Table 4]**

| Compound Tested | Example 1 | | Compound X |
|---|---|---|---|
| Compound Measured | Example 1 | Compound X | Compound X |
| AUC_{0→∞}(ng·h/m)) | not detected | 3439 ± 571 | 278 ± 104 |
| BA(%) | | 159 | 13 ± 5 |

As shown above, it was noted that the AUC calculated from concentration of the compound X in plasma when the compound of Example 1 was orally administered increased to an extent of 12-fold or more as compared with the AUC value when the compound X was administered. In addition, BA value calculated by the sum of concentrations of the compound of the present invention and of the compound X in plasma upon administration of the compound of Example 1 was calculated to be 159% and it was noted that there was a significant increase as compared with 13% which was the BA value upon administration of the compound X. From the above results, it was confirmed that, in the case of cynomolgus monkeys, the compound of the present invention also showed better pharmacokinetics as compared with the compound X.

### [Best Mode for Carrying Out the Invention]

The present invention will be further illustrated as follows.
In the present specification, the term "lower" means a linear or branched carbon chain having 1 to 6 carbon(s) unless otherwise stipulated.
Therefore, "lower alkyl" means C₁₋₆ linear or branched alkyl and its specific examples are methyl, ethyl, propyl, butyl, pentyl, hexyl, isopropyl and tert-butyl, etc. Preferably, it is C₁₋₄ linear or branched alkyl and, more preferably, methyl or ethyl.
"Lower alkenyl" means C₂₋₆ linear or branched alkenyl and its specific examples are ethenyl, propenyl, butenyl, pentenyl and hexenyl, etc. Preferably, it is C₂₋₃ linear or branched alkyl, i.e. ethenyl, 1-propenyl, 2-propenyl and 3-propenyl.
"Lower alkynyl" means C₂₋₆ linear or branched alkynyl and its specific examples are ethynyl, propynyl, butynyl, pentynyl and hexynyl, etc. Preferably, it is C₂₋₃ linear or branched alkynyl, i.e. ethynyl, 1-propynyl and 3-propynyl.
"Cycloalkyl" means a univalent group of C₃₋₁₀ carbon ring and it may partially have unsaturated bond and includes a bridged ring as well. Therefore, its specific examples are cyclopropyl, cyclobutyl, cyclopentyl, cyclohexyl, cycloheptyl, cyclooctyl, cyclobutenyl, cyclopentenyl, cyclohexenyl, cyclooctadienyl, bornyl, norbornyl, bicyclo[3.1.1]heptyl, bicyclo[2.2.2]octyl, bicyclo[3.2.1]octyl, bicyclo[3.3.1]nonyl, norbornenyl and adamantyl, etc. Preferably, it is cyclopentyl, cyclohexyl, cyclopentenyl or cyclohexenyl.
"Aryl" means a univalent monocyclic to tricyclic C₆₋₁₄ aromatic hydrocarbon ring and its specific examples are phenyl and naphthyl, etc. Preferably, it is phenyl.
"Non-aromatic hetero ring" means a univalent group of three- to eight-membered non-aromatic hetero ring containing one or more hetero atom(s) selected from the group consisting of nitrogen, oxygen and sulfur as ring-constituting atom(s) and it may partially contain unsaturated bond and includes a bridged ring as well. It may be also fused with phenyl. When sulfur is contained as a ring-constituting atom, the sulfur atom may be oxidized. Its specific examples are azetidinyl, pyrrolidinyl, imidazolinyl, imidazolidinyl, pyrazolidinyl, pyrazolinyl, piperidinyl, azepinyl, piperazinyl, homopiperazinyl, morpholinyl, thiomorpholinyl, indolinyl, isoindolinyl, dihydropyridyl, dihydrofuryl, tetrahydroquinolyl, tetrahydroisoquinolyl, tetrahydropyranyl, tetrahydrothiopyranyl, 1-oxidotetrahydrothiopyranyl, 1,1-dioxidotetrahydrothiopyranyl, quinuclidinyl and 8-azabicyclo[3.2.1]octyl, etc.
"Aromatic hetero ring" means a univalent group of five- to six-membered aromatic hetero ring containing one or more hetero atom(s) selected from the group consisting of nitrogen, oxygen and sulfur as ring-constituting atom(s) and it may be fused with phenyl. Its specific examples are pyridyl, pyrazyl, pyrimidinyl, pyridazinyl, furyl, thienyl, thiazolyl, oxazolyl, imidazolyl, indolyl, quinolyl, isoquinolyl, benzofuryl, benzothienyl and benzothiazolyl, etc.
With regard to acceptable substituent(s) for lower alkyl, lower alkenyl, lower alkynyl, cycloalkyl, aryl, non-aromatic hetero ring and aromatic hetero ring each of which is optionally substituted in -B, any group may be used so far as it is a substituent which is commonly used as a substituent for each group and each group may have one or more substituent(s).
With regard to acceptable substituent(s) for cycloalkyl, aryl, non-aromatic hetero ring and aromatic hetero ring each of which is optionally substituted in -B, groups which are shown in the following (a) to (h) may be exemplified. Here, "R^{Z}" means lower alkyl which may be substituted with one or more group(s) selected from the group consisting of -OH, -O-lower alkyl, amino which may be substituted with one or two lower alkyl(s) (when it is substituted with two lower alkyls, it may be substituted with either the same lower alkyl or may be substituted with different lower alkyls), aryl, aromatic hetero ring and halogen (hereinafter, the above definition will be applied as well).
(a) halogen;
(b) -OH, -O-R^{z}, -O-aryl, -OCO-R^{z}, oxo (=O);
(c) -SH, -S-R^{z}, -S-aryl, -SO-R^{z}, -SO-aryl, -SO₂-R^{z}, -SO₂-aryl, sulfamoyl which may be substituted with one or two R^{z} (when it is substituted with two R^{z}, it may be substituted with the same R^{z} or may be substituted with different R^{z});
(d) amino which may be substituted with one or two R^{z} (when it is substituted with two R^{z}, it may be substituted with the same R^{z} or may be substituted with different R^{z}), -NHCO-R^{z}, -NHCO-aryl, -NHCO₂-R^{z}, -NHCO₂-aryl, -NHSO₂-R^{z}, -NHSO₂-aryl, -NHCONH₂, -NHSO₂NH₂, nitro;
(e) -CHO, -CO-R^{z}, -CO₂H, -CO2-R^{z}, carbamoyl which may be substituted with one or two R^{z}, cyano;
(f) aryl or cycloalkyl which may be substituted with one or more group(s) selected from the group consisting of -OH, -O-lower alkyl, amino which may be substituted with one or two lower alkyl(s) (when it is substituted with two lower alkyls, it may be substituted with either the same lower alkyl or may be substituted with different lower alkyls), halogen and R^{z};
(g) aromatic hetero ring or non-aromatic hetero ring which may be substituted one or more groups selected from the group consisting of -OH, -O-lower alkyl, amino which may be substituted with one or two lower alkyl(s) (when it is substituted with two lower alkyls, it may be substituted with either the same lower alkyl or may be substituted with different lower alkyls), halogen and R^{z}; and
(h) lower alkyl, lower alkenyl or lower alkynyl which may be substituted with one or more groups selected from the substituents shown in the above (a) to (h).
With regard to an acceptable substituent for optionally substituted lower alkyl, lower alkenyl and lower alkynyl in -B, the groups shown in the above (a) to (g) may be exemplified.

Depending upon the type of the substituent, the compound of the present invention represented by the formula (I) may contain asymmetric carbon atom and there may be optical isomer due to that. The present invention includes all of those optical isomers both as a mixture thereof and as isolated ones. In some cases, tautomers may be present in the compound of the present invention and the present invention includes all of those tautomers both as isolated ones and as a mixture thereof. In addition, a labeled substance or, in other words, a compound in which one or more atom(s) of the compound of the present invention is/are substituted with radioisotope or non-radioactive isotope is included in the present invention as well.

. In some cases, the compound of the present invention forms a salt and, so far as such a salt is pharmaceutically acceptable, that is included in the present invention. Its specific examples are salt with inorganic acid such as hydrochloric acid, hydrobromic acid, hydroiodic acid, sulfuric acid, nitric acid and phosphoric acid, etc; an acid-addition salt with organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid and glutamic acid, etc; salt with an inorganic base including sodium, potassium, calcium or magnesium or with an organic base such as methylamine, ethylamine, ethanolamine, lysine or ornithine, etc; and ammonium salt, etc. The present invention further includes various kinds of hydrate and solvate of the compound of the present invention and a pharmaceutically acceptable salt and a substance having crystal polymorphism as well.

The compound of the present invention and a pharmaceutically acceptable salt thereof may be produced by applying various known synthetic methods utilizing the characteristic depending upon its fundamental skeleton or type of the substituent. Representative production processes will be exemplified as hereunder. Depending upon the type of a functional group, it is sometimes effective in view of manufacturing technique that the functional group is substituted with an appropriate protective group or, in other words, with a group which is easily able to be converted to the functional group, in a state from a staring material to an intermediate,. After that, the protective group is removed upon necessity whereupon an aimed compound is able to be produced. With regard to such a functional group, the functional groups which are exemplified, for example in "Protective Groups in Organic Synthesis (Third Edition)" by Greene and Wuts may be exemplified and they may be appropriately used depending upon the reaction conditions.

### (First production process)

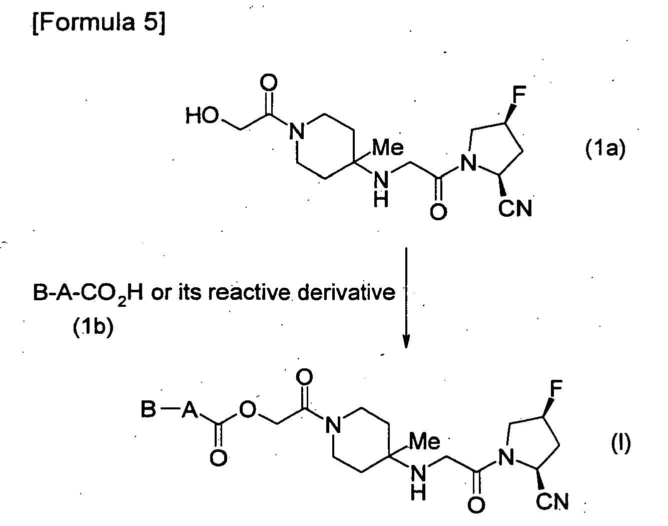

(In the formula, -A- and -B have the same meanings as defined already. They are the same hereinafter as well.)
This production process is a process for the production of the compound (I) of the present invention by acylation, making into carbonate, making into thiocarbonate or making into carbamate by a conventional method using Example 34 mentioned in WO 2004/009544 (Patent Document 8) or a free substance thereof or a salt of the free substance as a starting material.

Examples of the reactive derivative of the compound (1b) are acid halide such as acid chloride and acid bromide; acid azide; active ester such as ester with N-hydroxybenzotriazole, p-nitrophenol and N-hydroxysuccinimide; symmetric acid anhydride; common ester such as methyl ester, ethyl ester and tert-butyl ester; alkyl halocarboxylate such as alkyl carbonic acid halide; and mixed acid anhydride with pivaloyl halide, p-toluenesulfonyl chloride, etc. It is also possible to apply the method mentioned in the above "Protective Groups in Organic Synthesis (Third Edition)".
Particularly in the present invention, a method where acylation is conducted using an acid halide, a method where carbonate is produced using haloformate, a method where thiocarbonate is produced using formic acid thioester or a method where carbamate is produced using halocarbamate are suitable since they are able to produce the compound of the present invention easily.

Although being different depending upon the reactive derivative, etc. used, the reaction may be carried out with cooling, with cooling to at room temperature and/or at room temperature to with heating in an organic solvent which is inert to the reaction such as organic fatty acid solvent (e.g., acetic acid), ether (e.g., ether, tetrahydrofuran and dioxane), aprotic polar solvent (e.g., N,N-dimethylformamide and dimethyl sulfoxide) and halogenated hydrocarbon (e.g., methylene chloride, chloroform and dichloroethane). Depending upon the type of the reactive derivative used, it is also effective to use an organic acid corresponding to the acid halide used as a solvent.
In some cases, it is advantageous to smoothly proceed the reaction when, in conducting the reaction, the compound (1b) or a reactive derivative thereof is used excessively or when the reaction is conducted in the presence of a base such as triethylamine, diisopropyl ethyl amine, N-methylmorpholine, 4-(N,N-dimethylamino)pyridine and pyridine. Further, in order to prevent acylation, making into carbonate, making into thiocarbonate or making into carbamate of an amino group substituted at 4-position of piperidine in the compound (1a), it is sometimes preferred that the compound (1a) in its acid addition salt is used depending upon the type of the compound (1b) to be made to react.

### (Second production process)

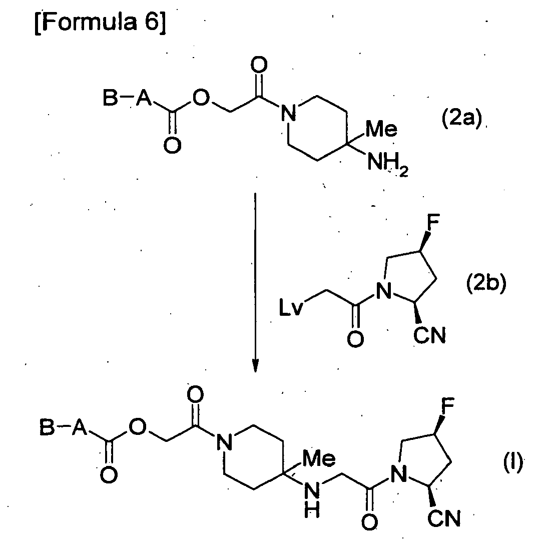

(In the formula, Lv is a leaving group such as halogen and sulfonyloxy.)
This producing process is a process for the production of the compound of the present invention (I) by alkylation of an amine (2a) with the compound (2b).

With regard to a leaving group represented by Lv, any group may be used so far as it is a leaving group which is commonly used for alkylation of an amine and its specific examples are halogen such as chlorine, bromine and iodine; and sulfonyloxy such as methanesulfonyloxy, ethanesulfonyloxy, trifluoromethanesulfonyloxy, benzenesulfonyloxy and p-toluenesulfonyloxy.
The reaction may be carried out with or without solvent and, in case that a solvent is used, the reaction may be carried out with cooling, with cooling to at room temperature and/or at room temperature to with heating in a solvent which is inert to the reaction such as aromatic hydrocarbon (e.g., toluene and xylene), ketone (e.g., methyl ethyl ketone and acetone), ether, alcohol (e.g., methanol, ethanol and 2-propanol), halogenated hydrocarbon, acetonitrile, aprotic polar solvent, water or a mixed solvent thereof.
In the present invention, addition of a base is useful for a smooth progress of the reaction. Specific examples of the base are an alkali carbonate such as sodium carbonate and potassium carbonate; an alkali hydrogen carbonate such as sodium hydrogen carbonate and potassium hydrogen carbonate; and an organic amine such as triethylamine, diisopropyl ethylamine, N-methylmorpholine, 4-(N,N-dimethylamino)pyridine and pyridine.

The compound of the present invention produced as such is isolated and purified as in its free form or as a salt thereof by subjecting to a salt-formation treatment by a known method. Isolation and purification are carried out by applying common chemical operations such as extraction, concentration, distillation, crystallization, filtration, recrystallization and various chromatographic means.
When the compound of the present invention has an asymmetric carbon, optical isomer is present. The optical isomer as such is able to be resolved by a common method such as a fractional crystallization where recrystallization is conducted with an appropriate salt and column chromatography. An optically active compound is also able to be produced using an appropriate optically active material.

The drug of the present invention is able to be prepared by a commonly used method using one or more compound(s) of the present invention represented by the formula (I) and a pharmaceutical carrier, an excipient and other additives which are commonly used for the manufacture of pharmaceutical preparations. Administration may be in any form of oral administration using tablet; pill, capsule, granule, diluted powder, liquid, etc. and of parenteral administration using injection such as intravenous injection and intramuscular injection or suppository or by nasal, mucosal or cutaneous route.

With regard to a solid composition for oral administration according to the present invention, there may be used tablet, diluted powder, granule, etc. In such a solid composition, one or more active ingredient(s) is/are mixed with at least one inert diluent such as lactose, mannitol, glucose, hydroxypropyl cellulose, microcrystalline cellulose, starch, polyvinylpyrrolidone and magnesium aluminate metasilicate. The composition may contain additives other than an inert diluent according to a conventional method such as lubricant (e.g., magnesium stearate), disintegrating agent (e.g., calcium cellulose glycolate), stabilizer and dissolving aid. If necessary, the tablet or pill may be coated with sugar coat or gastric or enteric film such as sucrose, gelatin, hydroxypropyl cellulose and hydroxypropyl methylcellulose phthalate.

A liquid composition for oral administration includes pharmaceutically acceptable emulsion, solution, suspension, syrup, elixir, etc. and contains commonly used inert diluent such as pure water and ethanol (EtOH). In addition to the inert diluent, the composition may also contain moisturizer, adjuvant such as suspending agent, sweetener, flavor, aromatizer and antiseptic.
Injection for parenteral administration includes aseptic aqueous or non-aqueous solution, it includes suspension and emulsion. Aqueous solution and suspension may contain distilled water for injection and physiological saline for example. Examples of non-aqueous solution and suspension are propylene glycol, polyethylene glycol, plant oil such as olive oil, alcohol such as EtOH and Polysolvate 80. Such a composition may further contain adjuvant such as antiseptic, moisturizer, emulsifier, dispersing agent, stabilizer and dissolving aid. They are sterilized by, for example, filtration through a bacteria-retaining filter or compounding or irradiation of bactericide. They may be also used in such a manner that an aseptic solid composition is manufactured and, before use, it is dissolved in an aseptic water or in an aseptic solvent for injection.

In the case of a common oral administration, it is appropriate that the daily dose is about 0.0001 to 50 mg/kg, preferably about 0.001 to 10 mg/kg or, more preferably, about 0.01 to 1 mg/kg body weight and it is administered once or divided into two to four times in a day to administer. In the case of an intravenous administration, it is appropriate that a daily dose is about 0.0001 to 1 mg/kg or, preferably, about 0.0001 to 0.1 mg/kg body weight and it is administered once or divided into plural times in a day to administer. The dose is appropriately decided depending upon each case by taking symptom, age, sex, etc. into consideration.

As hereunder, the present invention will be specifically illustrated by way of Examples although the present invention is not limited by those Examples at all.

### Examples

### Example 1

To a solution of 503 mg of (2S,4S)-4-fluoro-1-{[(1-glycoloyl-4-methylpiperidin-4-yl)amino]acetyl}pyrrolidine -2-carbonitrile monohydrochloride in 10 ml of acetic acid was added 0.3 ml of acetyl chloride followed by stirring at room temperature for 3 hours After the reaction solution was concentrated, the residue was dissolved in methanol and 28% aqueous ammonia (= 10:1), and 7 ml of silica gel was added to the solution followed by concentration *in vacuo.* The residue was subjected to silica gel column chromatography (eluent; chloroform : methanol : 28% aqueous ammonia = 30:1:0.1) to give 410 mg of a colorless amorphous. The resulting colorless amorphous was dissolved in 50 ml of methanol and 123 mg of fumaric acid was added thereto followed by concentration. To the residue were added methanol and ether and the resulting crystals were collected by filtration to give 317 mg of 2-[4-({2-[(2S,4S)-2-cyano-4-fluoropyrrolidin-1-yl]-2-oxoethyl}amino)-4-methylpi peridin-1-yl]-2-oxoethyl acetate monofumarate as colorless crystals.
FAB-MS [M + H]⁺: 369
¹H-NMR (DMSO-d₆): 1.05-1.15 (3H, s), 1.30-1.70 (4H, m), 2.07 (3H, s), 2.30-2.70 (2H, m), 3.20-4.10 (8H, m), 4.75 (2H, s), 4.90-5.05, 5.30-5.60 (2H, m), 6.59 (2H, s).

Other structures of the present invention are shown in the following Table 5 and 6. They may be easily produced by the above-mentioned production method, a method mentioned in Examples, a method which is obvious for persons skilled in the art or a modification thereof.
Symbols in the table have the following meanings.
No: compound No.; Me: methyl; Et: ethyl; nPr: n-propyl; iPr: isopropyl; nBu: n-butyl; iBu: isobutyl; tBu: tert-butyl; Ph: phenyl; cPen: cyclopentyl; cHex: cyclohexyl; Py: pyridyl; fur: furyl; the: thienyl; pipe: piperidin-4-yl; Ac: acetyl; Ms: methanesulfonyl; cyano: cyano; di: di. Figure(s) before a substituent show(s) substituted position(s) and, for example, 3,4-diF-Ph is 3,4-difluorophenyl, 1-Me-pipe is 1-methylpiperidin-4-yl and 3-the-O- is thiophen-3-yloxy.

**[Table 5]**

| | | | |
|---|---|---|---|
| 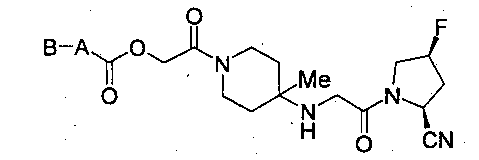 | | | |

| No | B-A- | No | B-A- |
|---|---|---|---|
| A1 | Et- | A2 | nPr- |
| A3 | iPr- | A4 | nBu- |
| A5 | iBu- | A6 | tBu- |
| A7 | MeO-CH₂- | A8 | EtO-CH₂- |
| A9 | F-(CH₂)₂- | A10 | HO-(CH₂)₂- |
| A11 | cyano-(CH₂)₂- | A12 | O₂N-(CH₂)₂- |
| A13 | H₂N-(CH₂)₂- | A14 | AcHN-(CH₂)₂- |
| A15 | MsHN-(CH₂)₂- | A16 | CH₂=CH-CH₂- |
| A17 | cPen- | A18 | cHex- |
| A19 | 4-HO-cHex- | A20 | 4-F-cHex- |
| A21 | cyclopenten-3-yl | A22 | Ph- |
| A23 | 4-Cl-Ph- | A24 | 4-F-Ph- |
| A25 | 3,4-diF-Ph- | A26 | 4-HO-Ph- |
| A27 | 4-H₂N-Ph- | A28 | 2-Ph-Ph- |
| A29 | naphthalen-2-yl | A30 | 4-Py- |
| A31 | 3-fur- | A32 | 3-the- |
| A33 | 1-Me-pipe- | A34 | 1-Et-pipe- |
| A35 | 1-(F-(CH₂)₂)-pipe- | A36 | 1-(HO-(CH₂)₂)-pipe- |
| A37 | 1-Ac-pipe- | A38 | 1-Ms-pipe- |
| A39 | 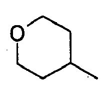 | A40 | 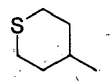 |
| A41 | 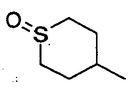 | A42 | 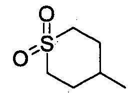 |

**[Table 6]**

| | | | |
|---|---|---|---|
| 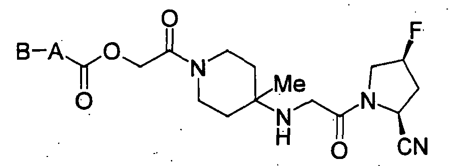 | | | |

| No | B-A- | No | B-A- |
|---|---|---|---|
| A43 | adamantan-1-yl | A44 | adamantan-2-yl |
| A45 | quinuclidin-3-yl | A46 | quinuclidin-4-yl |
| A47 | Me-O- | A48 | Et-O- |
| A49 | nPr-O- | A50 | iPr-O- |
| A51 | nBu-O- | A52 | tBu-O- |
| A53 | F-(CH₂)₂-O- | A54 | HO-(CH₂)₂-O- |
| A55 | CH2=CH-CH₂-O- | A56 | cHex-O- |
| A57 | Ph-O- | A58 | 4-Py-O- |
| A59 | 3-the-O- | A60 | 1-Ms-pipe-O- |
| A61 | Me-S- | A62 | Et-S- |
| A63 | iPr-S- | A64 | F-(CH₂)₂-S- |
| A65 | HO-(CH₂)₂-S- | A66 | cHex-S- |
| A67 | Ph-S- | A68 | 4-Py-S- |
| A69 | Me-NH- | A70 | Et-NH- |
| A71 | iPr-NH- | A72 | F-(CH₂)₂-NH- |
| A73 | HO-(CH₂)₂-NH- | A74 | cHex-NH- |
| A75 | Ph-NH- | A76 | 4-Py-NH- |
| A77 | Me-N(Me)- | A78 | Et-N(Me)- |
| A79 | iPr-N(Me)- | A80 | F-(CH₂)₂-N(Me)- |
| A81 | HO-(CH₂)₂-N(Me)- | A82 | cHex-N(Me)- |
| A83 | Ph-N(Me)- | A84 | 4-Py-N(Me)- |

### [Industrial Applicability]

The compound of the present invention has an excellent DPP-IV inhibitory activity and also shows an excellent pharmacokinetics. Accordingly, based upon the action as such, the compound of the present invention is useful for treatment and/or prevention of insulin-dependent diabetes mellitus (type 1 diabetes mellitus) and particularly for non insulin-dependent diabetes mellitus (type 2 diabetes mellitus), insulin-resistant diseases, obesity, etc.

## Claims

1. A pyrrolidine derivative represented by the following formula (I) or a pharmaceutically acceptable salt thereof. 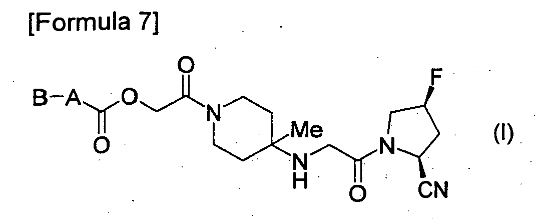 [In the formula, -A- is a single bond, -O-, -S-, -NH- or-N(lower alkyl)-; and -B is lower alkyl, lower alkenyl, lower alkynyl, cycloalkyl, aryl, non-aromatic hetero ring or aromatic hetero ring and each of those groups may be substituted.]

2. The compound according to claim 1, wherein -A- is a single bond.

3. The compound according to claim 2, wherein -B is an optionally substituted lower alkyl or an optionally substituted aryl.

4. The compound according to claim 3, wherein -B is an optionally substituted lower alkyl.

5. The compound according to claim 4, wherein -B is methyl.

6. The compound according to claim 1, wherein -A- is -O-.

7. The compound according to claim 6, wherein -B is an optionally substituted lower alkyl or an optionally substituted aryl.

8. The compound according to claim 7, wherein -B is an optionally substituted lower alkyl.

9. The compound according to claim 8, wherein -B is methyl.

10. A pharmaceutical composition which is comprising the compound mentioned in claim 1 as an active ingredient.

11. The pharmaceutical composition according to claim 10, wherein it is a treating agent for insulin-dependent diabetes mellitus (type 1 diabetes mellitus), insulin-independent diabetes mellitus (type 2 diabetes mellitus), insulin-resistant diseases or obesity.

12. The pharmaceutical composition according to claim 10, wherein it is a dipeptidyl peptidase IV inhibitor.
